# EUROPEAN PATENT APPLICATION

(11) **EP 1 266 963 A1**
(43) Date of publication of application: **18.12.2002**
(21) Application number: 01202304.0
(22) Date of filing: 15.06.2001
(51) Int. Cl.: C12N 15/10, G01N 33/68, C12N 7/00

(54) **Chimaeric phages**

(71) Applicant: Crucell Holland B.V., 2333 CN Leiden (NL)
(72) Inventor: Houtzager, Erwin, 3958 XD Amerongen (NL); Logtenberg, Ton, 3433 CH Niewuwegein (NL); de Kruif, Cornelis Adriaan, 3513 VN Utrecht (NL)
(74) Representative: Prins, Adrianus Willem

(57) **Abstract**

The invention is concerned with recombinant bacteriophages, particularly for display of proteinaceous substances. Means and methods are provided to optimise the selection of particular phages from a library of phages. To this end the invention in one embodiment provides a chimearic phage having a coat comprising a mixture of proteins. The mixture comprises at least a fusion protein wherein (a part of) a coat protein is fused to proteinaceous molecule and wherein said mixture further comprises a mutant of said coat protein.

## Description

### FIELD OF THE INVENTION

The present invention relates to the fields of molecular biology and immunology. The invention in particular relates to the field of generating helper phages and phage display libraries for the identification of binding molecules.

### BACKGROUND OF THE INVENTION

An individual needs to have a dynamic immune system that is able to adapt rapidly to respond adequately to potentially harmful microorganisms and to respond to the exposure of a highly diverse and continuously changing environment. Higher organisms have evolved specialized molecular mechanisms to ensure the implementation of clonally-distributed, highly diverse repertoires of antigen-receptor molecules expressed by cells of the immune system: immunoglobulin (Ig) molecules on B lymphocytes and T cell receptors on T lymphocytes. A primary repertoire of (generally low affinity) Ig receptors is established during B cell differentiation in the bone marrow as a result of rearrangement of germ line-encoded gene segments. Further refinement of Ig receptor specificity and affinity occurs in peripheral lymphoid organs where antigen-stimulated B lymphocytes activate a somatic hypermutation machinery that specifically targets the immunoglobulin variable (V) regions. During this process, B cell clones with mutant Ig receptors of higher affinity for the inciting antigen are stimulated into clonal proliferation and maturation into antibody-secreting plasma cells (reviewed in Berek and Milstein. 1987).

Recombinant DNA technology has been used to mimic many aspects of the processes that govern the generation and selection of natural human antibody repertoires (reviewed in Winter and Milstein. 1991; Vaughan et al. 1998). The construction of large repertoires of antibody fragments (such as Fab fragments or single chain Fv fragments, scFv's) expressed on the surface of filamentous phage particles and the selection of such phages by "panning" on antigens has been developed as a versatile and rapid method to obtain antibodies of desired specificities (reviewed in Burton and Barbas. 1994). A subsequent optimization of the affinity of individual phage antibodies was achieved by creating mutant antibody repertoires of the selected phages and sampled for higher affinity descendents by selection for binding to antigen under more stringent conditions (reviewed in Hoogenboom. 1994).

M13 and M13-derived phages (sometimes also called viruses) are filamentous phages that can selectively infect F-pili bearing (F⁺) *Escherichia coli* (*E.coli*) cells. The phage genome encodes 11 proteins, while the phage coat itself consists of 5 of these proteins: gene3, -6, -7, -8 and -9 (g3, g6, g7, g8 and g9) proteins that are bound to and that protect the (circular) single stranded DNA (ssDNA) of the viral genome. The life cycle of the virus can be subdivided into different phases. After infection of an *E.coli* by a phage particle, the ssDNA of the virus becomes double stranded due to the action of a number of bacterial enzymes. The double stranded phage genome now serves as a template for the transcription and translation of all 11 genes located on the phage genome. Besides these protein-encoding regions, the phage genome contains an intergenic region: the F1-origin of replication initiation (F1-ORI). The DNA sequence of this F1-ORI can be divided in 2 separate subregions. One subregion is responsible for the initiation and termination of the synthesis of ssDNA via the so-called "rolling circle mechanism" and the other subregion is responsible for the packaging initiation of the formed circular ssDNA leading to the formation and release of new virus particles.

It has been shown that polypeptides, such as stretches of amino acids, protein parts or even entire proteins can be added by means of molecular genetics to the terminal ends of a number of particle coat proteins, without disturbing the functionality of these proteins in the phage life cycle (Smith. 1985; Cwirla et al. 1990; Devlin et al. 1990; Bass et al. 1990; Felici et al. 1993; Luzzago et al. 1993).

This feature enables investigators to display peptides or proteins on phages, resulting in the generation of peptide- or protein expression phage display libraries. One of the proteins that has been used in the art to fuse with polypeptides for phage display purposes, is the g3 protein (g3p), which is a coat protein that is required for an efficient and effective infectivity and subsequent entry of the viral genome into the *E.coli* cell.

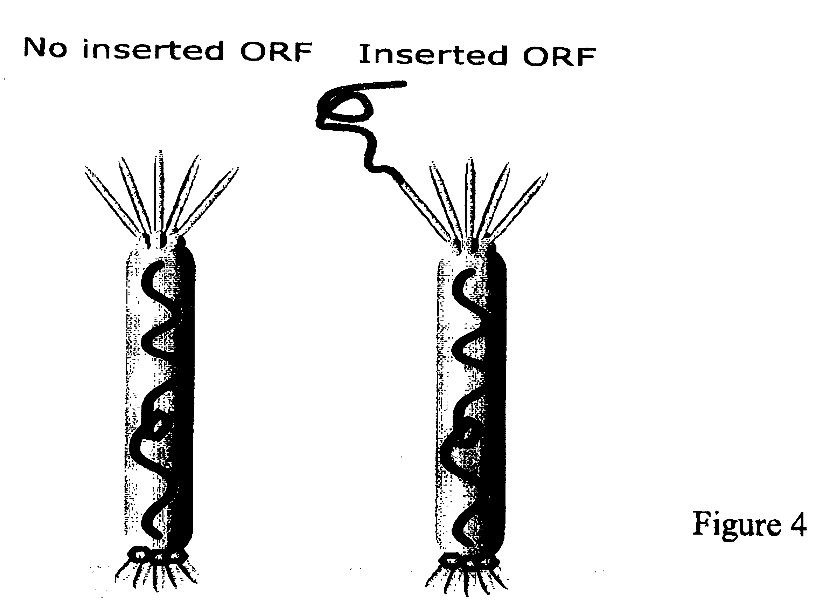

For the production of phages that display polypeptides fused to the g3p coat protein, investigators in the art introduced a plasmid together with the phage genome in *E.coli* cells. This plasmid contains an active promoter upstream of an in-frame fusion between the g3 encoding gene and a gene of interest (X) encoding, for instance, polypeptides such as proteins such as antibodies or fragments such as Fab fragments or scFv's. The introduction of this plasmid together with the genome of the helper phage in an *E.coli* cell results in the generation of phages that contain on their coat either the wild type g3p from the viral genome, the fusion product g3p-X from the plasmid or a mixture of the two, since one phage particle carries five g3p's on its surface. The process of g3p or g3p-X incorporation is generally random. The presence of an F1-ORI sequence in the g3p-X expression vector (plasmid) misleads the phage synthesis machinery in such a way that two types of circular ssDNA are formed: one is derived from the genome of the phage and the other is derived from the expression vector. During the synthesis of new phages the machinery is unable to distinguish the difference between these two forms of ssDNA resulting in the synthesis of a mixed population of phages, one part containing the phage genome and one part harbouring the vector DNA. Due to these processes the mixture contains at least some phages in which the phenotypic information on the outside (the g3p-X fusion protein) is conserved within the genotypic information inside the particle (the g3p-X expression vector). An infectious wild type phage and a phage carrying a fusion protein attached to g3p are depicted in **Figure 4.** The art teaches that there are several problems that concern the use of these basic set-ups.

The high level of genotypic wild type phages in phage populations grown in bacteria that contain both the phage genome and the expression vector compelled investigators to design mutant F1-ORI sequences in M13 genomes. Such mutant M13-strains are less effective in incorporating their genome in phage particles during phage assembly, resulting in an increased percentage of phages containing vector sequences when co-expressed. These mutant phages, such as the commercially available strains R408, VCSM13 and M13KO7, are called "helper phages". The genome of these helper phages may contain genes required to assemble new (helper-) phages in *E.coli* and to subsequently infect new F-pili expressing *E.coli.* Both VCSM13 and M13K07 were provided with an origin of replication initiation (ORI) of the P15A type that results in the multiplication of the viral genome in *E.coli.* Moreover, the ORI introduction ensures that after cell division the old and newly formed *E.coli* contains at least one copy of the viral genome.

It was suggested and finally proven by several investigators that the introduction of plasmids containing a g3p-scFv fusion product together with the genome of the helper phages in *E.coli* cells results in approximately 99% of newly formed phages that harbor the g3p-scFv fusion protein expression plasmid but nevertheless lack the g3p-scFv fusion on its surface (Beekwilder et al. 1999). The absence of g3p-X is a significant disadvantage in the use of display libraries for the identification of specific proteins or peptides such as scFv's that bind to a target of interest (such as tumor antigens). It implies that in the case of phage display libraries at least a 100-fold excess of produced phages must be used in an experiment in order to perform a selection with all possible fusion proteins present. The art teaches that this overload of relatively useless phages in an experiment leads to (too) many false positives. For instance, at least 10¹² phages should be added to a panning experiment in order to have 1 copy of each possible fusion present in the experiment, since such a library contains approximately 10¹⁰ different g3p-scFv fusions (1%). The phages in this approximate 1% express generally only 1 g3p-scFv fusion on their coat together with four normal g3p's (no fusions), while the rest of the helper phages (approximately 99%) express five g3p's and no g3p-scFv fusions. To ensure, theoretically, the presence of 100 copies of each separate fusion protein in a panning experiment, one needs to use approximately 10¹⁴ phages in such an experiment. Persons skilled in the art generally attempt to use an excess of at least 100-fold of each single unique fusion protein, to ensure the presence of sufficient numbers of each separate fusion and not to lose relevant binders too quickly in first panning rounds. That number of phages (10¹⁴) is more or less the maximum of phage particles that one millilitre (ml) can hold. The viscosity of such a solution is extremely high and therefore relatively useless. Especially when ELISA panning strategies are used (in which the volume of one well is only 200 µl) such libraries cannot be used.

In addition to these problems, it is shown that, depending on the antigen, an average of 1 in every 10⁷ phages will bind to the antigen due to a-specific binding. Generally as mentioned, for the addition of 10¹² scFv expressing input phages (1%) to a panning procedure, one has to add approximately 10¹⁴ phages (99% does not express a scFv fragment). It is generally assumed that from these 10¹² phages approximately 10⁴ particles might be putatively interesting phages. However, the number of calculated background phages that are normally found by using libraries present in the art after one round of panning, was approximately 10⁶-10⁷ while only a few of these phages appear to be relevant binders. This is one of the most significant problems recognized in the art: too many background phages show up as initial binders in the phage mix after the first round of panning, while only a few significant and interesting binders are present in this mix. So, the absolute number of isolated phages after one round of panning is clearly too high (10⁶-10⁷). Moreover, also in subsequent rounds of panning non-specific background phages remain present. In libraries used in the art, most of these non-specific binders will amplify on bacteria upon amplification to continue appearing in a second round of panning. Therefore, the art teaches that the background level of a-specifically binding phages and the total number of phages per ml in these types of libraries is unacceptably high and remains high during subsequent rounds of panning.

A possibility that was suggested by investigators in the art as a solution to the problem of obtaining too many background phages that lack a g3p-X fusion was to remove the g3p-encoding gene entirely from the helper phage genome. In principle, this system ensures that during phage synthesis in an *E.coli* cell (that received the g3-less phage genome and a g3p-X fusion protein expression vector) only g3p-X proteins are incorporated in the newly formed phage coat. By doing so, each synthesized phage will express five copies of the g3p-X fusion product and hardly any phages are synthesized that express the g3p alone or that express less than five g3p-X fusions. R408-d3 and M13ΔD3 are two examples of g3-minus helper phages (Dueòas and Borrebaeck. 1995; Rakonjac et al. 1997). Because the genome of these phages is not capable of supporting g3p synthesis, phage particles that carry less than five g3p-X fusion proteins can hardly be formed, or, if formed, are found to be non-infectious due to instability, since the art teaches that five g3p's are necessary to ensure a stable phage particle.

To produce helper phages that do not contain the g3 gene, but that are nevertheless infectious and that can be used to generate libraries of phages that carry five g3p-X fusion proteins, and that lack phages with less than five g3p-X fusions, it was recognized in the art that an external source for g3p was required. Such a source can be a vector without F1-ORI but that nevertheless contains an active promoter upstream of the full open reading frame (ORF) of g3. One major problem that is recognized by persons skilled in the art is that after the generation step of producing newly formed helper phages lacking a g3 gene, the yield is dramatically low. In fact, the yield of all described systems is below 10¹⁰ phages per liter, meaning that for a library of 10¹⁰ individual clones at least 100 liter of helper phage culture is necessary (NB: the helper phages need to be purified) in order to grow the library once. The art thus teaches that phage libraries generated with such low titers of helper phages are not useful for phage display purposes and that therefore these libraries cannot be used for panning experiments.

Phages that express deleted g3p's fused to heterologous proteins have also been generated. For the construction of most conventional Fab libraries and some scFv phage display libraries the D1 domain and parts of the D2 domain were removed to ensure a shorter fusion protein, which was considered in the art as a product that could be translated easier than a full length g3p linked to a full length Fab fragment. The shorter g3p part would not prevent the generation of a viable and useful helper phage. Of course, such phages still depend on full length g3p's that are present on their surface next to the deleted g3p fusion with the Fab fragment for functional infectivity of *E.coli* cells. Also, phages that express deleted g3p's fused to ligand-binding proteins have been generated that depend on their infectious abilities on antigens that were fused to the parts of g3p that were missing from the non-infectious phage particle (reviewed by Spada et al. 1997). These particles depend for their infectivity on an interaction between the ligand-binding protein and their respective ligand.

The g3-minus helper phages R408-d3 and M13ΔD3 mentioned above, lack in their genome a bacterial ORI and a selection marker. The absence of a selection marker in the g3-minus genomes has a significant effect on the production scale of helper phages, because it results in an overgrowth of bacteria that do not contain the helper phage genome. It is known that bacteria grow slower when infected with the helper phage or virus. Therefore, bacteria that lack the phage genome quickly overgrow the other bacteria that do contain the genome. Another effect of the lack of an ORI or a selection marker is that g3-minus phage genomes cannot be kept in dividing bacteria during the production and expansion of phage display libraries. This is a very important negative feature because overgrowth of bacteria that lost the phage genome or that did never receive one, appear to have a growth advantage over bacteria that do contain the phage genome. In addition of course, such 'empty' bacteria are not capable of producing any phage and as a result of this the phage display vectors including fusion protein fragments in such helper phages lacking-bacteria are lost permanently.

As mentioned, the g3p's are thought to be essential for the assembly of stable M13-like phages and because of their crucial role in infection, g3p's should be provided otherwise when g3-minus helper phages are to be generated. There is a prejudice in the art against making phage display libraries that lack g3p's because phages lacking g3p's are not stable. Rakonjac et al. (1997) constructed a VCSM13 g3-minus helper phage in parallel to a R408 g3-minus helper phage and used helper plasmids with either the psp or the lac promoter upstream of a full length g3 sequence to substitute g3 during helper phage synthesis (Model et al. 1997). However, the art teaches that the lac promoter has the disadvantage that it cannot be shut off completely, even not in the presence of high concentrations of glucose (3-5%) in the medium (Rakonjac and Model. 1998). An additional problem that is well known in the art is that even very low levels of g3p in *E.coli* can block infection of M13-like phages. Moreover, it has been shown that co-encapsidation of plasmids together with the phage genome can occur (Russel and Model. 1989; Krebber et al. 1995; Rakonjac et al. 1997). If co-encapsidation occurs with the lac driven helper plasmid it will compete with the lac driven vectors used in the phage display resulting in the efficient production of infectious phage particles that will not contain the g3p-X fusion product. Together, the art thus teaches that the lac promoter is not the best candidate promoter in the helper plasmid system. The psp promoter has the advantage to be relatively silent in *E.coli* until infection (Rakonjac et al. 1997). Upon M13-class phage infection the psp promoter becomes activated and now the helper plasmid will produce g3 proteins. However, the disadvantage of this promoter is that the level of RNA production cannot be regulated with external factors, but has to be regulated by either mutating (and change the activity of) the promotor, changing the ribosomal binding site (RBS) or other elements that influence the promotor activity. To figure out the ideal level of promotor activity in a specific *E.coli* strain can be time consuming and needs to be optimized for each *E.coli* strain separately. The art also teaches that the psp promotor system is not very attractive for large-scale helper phage production due to the inflexibility of *E.coli* strains, the time consuming optimization and the significant low level of helper phage production.

One other significant problematic feature of all helper phage systems described is the occurrence of unwanted recombination events between the helper genome and the (helper-) plasmids. The problem that confronts investigators in the art is the fact that the g3 DNA sequences in the helper phages are homologous to the g3 sequences in the phage display vector and/or the helper phage plasmid. This results in many cases in recombination between the two DNA strains and therefore loss of functionality of the library as a whole.

It is an object of the present invention to deal with problems and drawbacks known from the art as described above, concerning the generation of phage particles and helper phages, the use of helper phages in the production of phage display libraries, and the problems and drawbacks known for the identification of relevant binding molecules using such libraries.

### SUMMARY OF THE INVENTION

The current invention comprises methods and means that substantially lack above outlined drawbacks and that are characterized in providing novel phage particles such as chimaeric phages, novel helper phages, libraries comprising said chimaeric phages and methods and means to produce said chimaeric phages and said helper phages.

The invention provides a chimaeric phage having a coat comprising a mixture of proteins, said mixture comprising a fusion protein wherein a proteinaceous molecule is fused to a functional form of a phage coat protein, said mixture further comprising a mutant form of said phage coat protein, wherein said mutant form is characterized in that a phage, comprising no wild type phage coat protein from which said mutant form is derived and having a coat comprising said mutant form and no copies of said functional form, is less infectious than a phage, comprising no wild type phage coat protein from which said mutant form is derived and having a coat comprising said mutant form and at least one copy of said functional form. In another embodiment of the invention, said mutant form is characterized in that a phage, comprising no wild type phage coat protein from which said mutant form is derived and carrying said mutant form and no copies of said fusion protein is less infectious than a phage, comprising no wild type phage coat protein from which said mutant form is derived and carrying in addition to said mutant form at least one copy of said fusion protein. Preferably, said mutant form is characterized in that a phage, comprising no wild type phage coat protein from which said mutant form is derived and carrying said mutant form and no copies of said fusion protein or said functional form is non-infectious. More preferably, said mutant form is further characterized in that a phage, having a coat comprising said mutant form in the presence or absence of copies of said functional form, is stable. Stable as use herein means that the part of g3p that is still present in said mutant form (and that is also present in said functional form) ensures features such as DNA binding and rigidness of the phage, but does not contribute to infectiousness of said phage as do the domains in said functional form that are not present in said mutant form. The invention also provides an infectious phage containing at least one copy of a mutant form of a phage coat protein, wherein said mutant form has lost the ability to mediate infection of a natural host by said infectious phage.

In a preferred embodiment, said phage coat protein is the g3 protein (g3p) present in the coat of phages such as M13 and R408, and said mutant form comprises a mutation in the D1 and/or the D2 region of g3p. In a preferred aspect of the invention said chimaeric phage or said infectious phage is part of a phage collection such as a phage display library. In a more preferred aspect of the invention such a phage collection consists essentially of chimaeric phages or infectious phages provided by the invention. Also preferred are chimaeric phages or infectious phages according to the invention that comprise antibodies or fragments thereof as part of said fusion protein.

In another embodiment the invention provides a method for producing a phage particle comprising the steps of providing a host cell with a first nucleic acid encoding a fusion protein, said fusion protein comprising a proteinaceous molecule fused to a functional form of a phage coat protein, providing said host cell with a second nucleic acid encoding a mutant form of said phage coat protein, said mutant form being characterized in that a phage, comprising no wild type phage coat protein from which said mutant form is derived and having a coat comprising said mutant form and no copies of said functional form is less infectious than a phage, comprising no wild type phage coat protein from which said mutant form is derived and having a coat comprising at least one copy of said functional form, and wherein said host cell comprises an additional nucleic acid sequence encoding at least all other proteins or functional equivalents thereof, that are essential for the assembly of said phage particle in said host cell, and culturing said host cell to allow assembly of said phage particle. Preferably, said method for producing a phage particle is applied for producing said chimaeric phage or said infectious phage. More preferably said method is applied for producing a phage particle such as a chimaeric phage or an infectious phage provided by the invention that comprise nucleic acid encoding said mutant form under the control of a controllable promoter such as the AraC/BAD promoter.

In another embodiment the invention provides a helper phage comprising nucleic acid encoding phage proteins or functional equivalents thereof that are essential for the assembly of said helper phage, said nucleic acid further encoding a mutant form of a phage coat protein, wherein said mutant form is characterized in that a phage, comprising no wild type phage coat protein from which said mutant form is derived and having a coat comprising said mutant form and no copies of a functional form of said phage coat protein, is less infectious than a phage, comprising no wild type phage coat protein from which said mutant form is derived and having a coat comprising at least one copy of said functional form, wherein said functional form is characterized in that it renders a phage particle carrying said functional form in its coat infectious, and wherein said helper phage does not comprise nucleic acid encoding said functional form.

In yet another embodiment the invention provides a method for producing a helper phage comprising the steps of providing a host cell with a first nucleic acid encoding a functional form of a phage coat protein, providing said host cell with a second nucleic acid encoding a mutant form of said phage coat protein, wherein said mutant form is characterized in that a phage, comprising no wild type phage coat protein from which said mutant form is derived and having a coat comprising said mutant form, is less infectious than a phage comprising no wild type phage coat protein from which said mutant form is derived and having a coat comprising at least one copy of said functional form, wherein said host cell comprises an additional nucleic acid sequence encoding at least all other proteins or functional equivalents thereof that are essential for the assembly of said helper phage in said host cell, and culturing said host cell to allow assembly of said helper phage.

In another aspect the invention provides methods and means for producing a phage particle such as a chimaeric phage, an infectious phage or a helper phage according to the invention, wherein separate nucleic acids encoding either (1) a functional form of said phage coat protein alone or fused to a proteinaceous molecule or (2) a mutant form of said phage coat protein, each comprise codons in the overlapping regions between the protein encoding parts, that essentially do not render a homologous recombination event between said separate nucleic acids. In a preferred aspect said separate nucleic acids each comprises non-interfering origins of replication and unique selection markers.

In another embodiment the invention provides a method for the enrichment of a first binding pair member in a repertoire of first binding pair members selected from the group consisting of an antibody, an antibody fragment, a single chain Fv fragment, a Fab fragment, a variable region, a CDR region, an immunoglobulin or a functional part thereof, said first binding pair member being specific for a second binding pair member, comprising the steps of contacting a phage collection comprising chimaeric or infectious phages according to the invention, with material comprising said second binding pair member under conditions allowing specific binding, removing non-specific binders, and recovering specific binders, said specific binders comprising said first binding pair member. Said material may comprise second binding pair members such as purified proteins, recombinant proteins and/or proteins present on cells. In a preferred embodiment, the invention provides a method for the enrichment of a first binding pair member that furthermore comprise the steps of recovering from a phage a DNA sequence encoding said first specific binding pair member, subcloning said DNA sequence in a suitable expression vector, and expressing said DNA sequence in a suitable host, and culturing said suitable host under conditions whereby said first specific binding pair member is produced. A suitable expression vector may be a plasmid vector comprising an active promoter that regulates the expression of said first specific binding pair member in suitable hosts like eukaryotic cells such as yeast cells or mammalian cells.

In another aspect, the invention provides a nucleic acid molecule comprising a sequence encoding a mutant form of a phage coat protein, said mutant form being characterized in that a phage, comprising no wild type phage coat protein from which said mutant form is derived and having a coat comprising said mutant form and no functional form of said phage coat protein, is less infectious than a phage comprising no wild type phage coat protein from which said mutant form is derived and having a coat comprising said mutant form and at least one copy of said functional form of said phage coat protein, wherein said functional form is characterized in that it renders a phage carrying said functional form in its coat infectious. Said nucleic acid molecule may furthermore comprise all relevant nucleic acid encoding proteins that are required for assembly of a phage in a host cell.

### DETAILED DESCRIPTION OF THE INVENTION

The invention provides a chimaeric phage having a coat comprising a mixture of proteins, said mixture comprising a fusion protein wherein a proteinaceous molecule is fused to a functional form of a phage coat protein, said mixture further comprising a mutant form of said phage coat protein, wherein said mutant form is characterized in that a phage, comprising no wild type phage coat protein from which said mutant form is derived and having a coat comprising a mutant form of said phage coat protein and no copy or copies of said functional form of said phage coat protein, is less infectious than a phage comprising no wild type phage coat protein from which said mutant form is derived and having a coat comprising at least one functional form of said phage coat protein. Functional form as used herein refers to a phage coat protein that contributes significantly to the infectiousness of the particle to which it is attached. The phage coat protein itself is not infectious, but the functional form renders the phage particle to which it is attached infectious. Besides the contribution to infectivity of the phage particle, the phage coat protein also sustains other functions such as stabilization of the phage particle. A mutant form of a phage coat protein according to the invention may render the phage particle less infectious or non-infectious but should still sustain other functions of said phage coat protein such as stabilization of the phage. A phage that comprises no wild type phage coat protein from which said mutant form is derived and comprises no functional forms of said phage coat protein but does contain only mutant forms of said phage coat protein is less infectious than a phage that comprises no wild type phage coat protein from which said mutant form is derived and comprises one or more functional forms of said phage coat protein next to the mutant forms of said phage coat protein in its coat. Less infectious as used herein may also mean non-infectious. Although a chimaeric phage of the invention comprises at least one copy of the mutant form of said phage coat protein in its coat, the chimaeric phage has infectious capability because it also comprises at least one functional form of said phage coat protein in its coat. A functional form of a phage coat protein as used herein also means a part, derivative and/or an analogue thereof that still harbors functionality in rendering the phage infectious to which it is attached. Mutant form of a phage coat protein as used herein also means a part, a derivative and/or an analogue of said mutant form, wherein said mutant form is characterized in that a phage, comprising no wild type phage coat protein from which said mutant form is derived and having a coat comprising only mutant forms of said phage coat protein or parts, derivatives and/or analogues thereof, is less- or non-infectious as compared to a phage comprising no wild type phage coat protein from which said mutant form is derived and having a coat comprising at least one functional form of said phage coat protein.

In a preferred embodiment said phage coat protein is the g3 protein (g3p) that in a wild type or functional form renders a phage to which it is attached, infectious. In another preferred embodiment the mutant form of said phage coat protein comprises an alteration in the g3 protein consisting of a mutation in either the D1 region, the D2 region or both. A mutation as used herein means one or multiple point mutations, stretches of mutations, deletions, substitutions, replacements and/or swapping of parts. In a more preferred embodiment said alteration in the g3 protein is a deletion of substantially all of the D1 and/or the D2 region. Said alteration may also mean a substitution of the deleted g3 protein part by a protein or a peptide not contributing to the infectivity of the helper phage, the chimaeric phage, the infectious phage or the phage particle.

In an even more preferred embodiment of the invention a chimaeric phage or an infectious phage according to the invention comprises a nucleic acid encoding a fusion protein, wherein a proteinaceous molecule is fused to a functional form of said phage coat protein and wherein said chimaeric phage of the invention comprises a M13, M13K07, VCSM13 or a R408 strain or a mutant, derivative or analogue strain derived from either one of these strains. A proteinaceous molecule according to the invention is fused to said functional form of said phage coat protein and comprises a protein such as a ligand-binding moiety or an immunoglobulin (such as an antibody). A proteinaceous molecule may also mean a peptide such as a random stretch of amino acids or a non-random stretch of amino acids such as an antibody fragment or derivatives thereof (Fab fragment, a single chain Fv fragment (scFv), a variable region or a CDR region). A proteinaceous molecule can also mean first specific binding pair member or can mean fusions between different kinds of (fragments of) proteins and/or fusions between (fragments of) proteins and (random and non-random) peptides such as antibody-recognized tags.

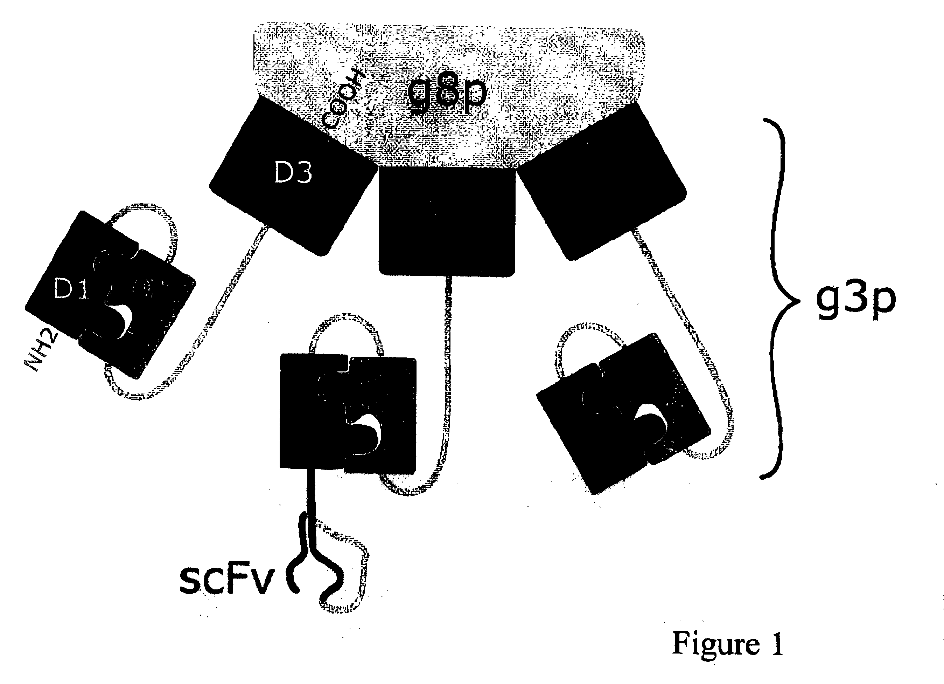

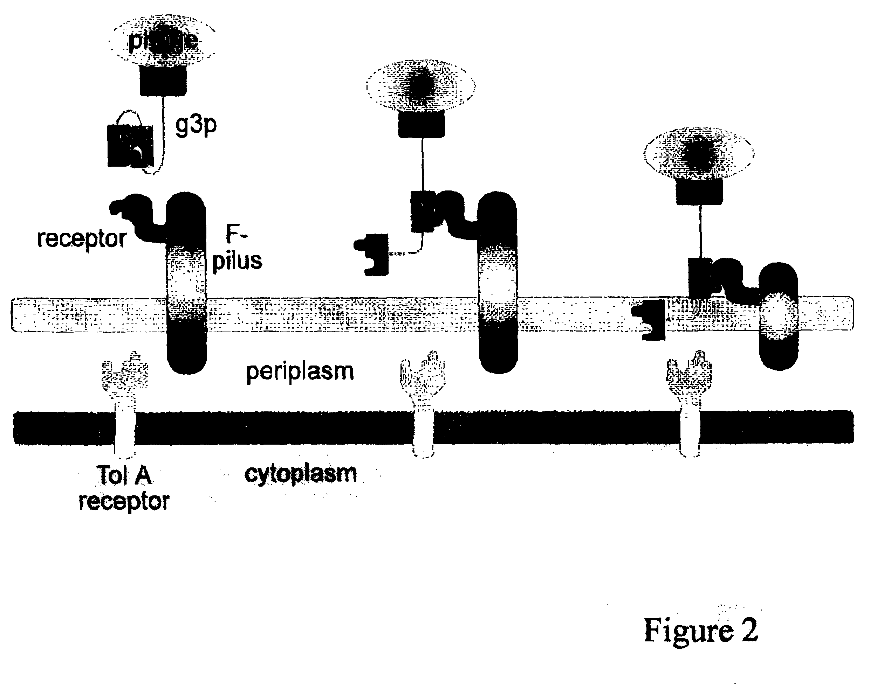

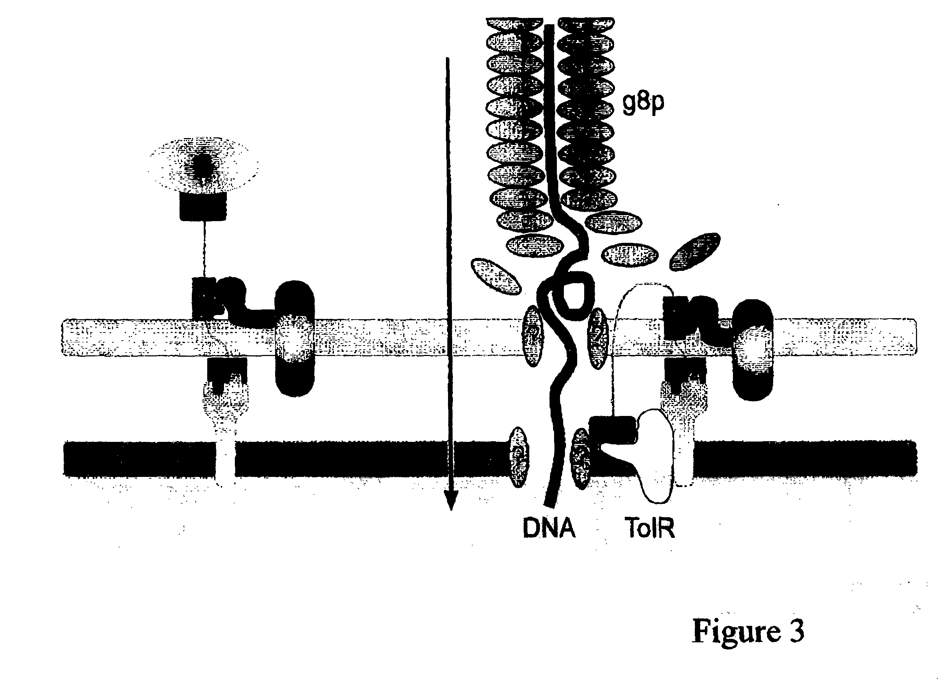

The g3 protein (g3p) of M13 phages and M13-derivatives comprises three functional domains: D1, D2 and D3, linked by two glycin-rich linkers. Considering that functionality of a D3 domain of the protein is required for assembly of stable phages, a less-, or non-infectious mutant of said phage coat protein preferably comprises a D3 region of said g3p, or comprises a functional part, derivative and/or analogue of said D3 region. The D3 domain is thought to bind to DNA inside the viral particle. Loss of the D3 domain functionally results in rare phage-like particles that are very long and very fragile (Pratt et al. 1969; Crissman and Smith. 1984; Rakonjac and Model. 1998). The D1 and D2 domain are thought to interact with each other until the phage binds to the bacteria. Studies in which a protease cleavage site was introduced between D1 and D2 showed that after cleavage the phage particle became non-infectious (Kristensen and Winter. 1998). Functional analysis of g3p showed that of the g3p N-terminal regions, the D1 domain is essential for infection. Loss of this domain results in phages that cannot infect bacteria (Lubkowski et al. 1998; Nelson et al. 1981; Deng et al. 1999; Riechmann and Holliger. 1997; Holliger and Riechmann. 1997). It has been shown that the D2 domain interacts with the D1 domain of g3p on the phage (**Figure 1**). Due to competition of proteins located on the F-pilus (on F⁺ bacteria) that have higher affinity for D2 than for D1, the D1 and D2 domain of the g3p dissociate from each other. The binding of D2 to the F-pilus results in a process that leads to retraction of the F-pilus towards the *E.coli* cell membrane. Due to this process the phage particle comes in close contact with the bacterial membrane. Now the dissociated D1 domain can interact with bacterial proteins such as the TolA receptor, leading to the introduction of the phage DNA into the *E.coli* cell (Lubkowski et al. 1999). The fact that removal of the D2 domain does not prevent infection but enables phages to infect *E.coli* lacking F-pili (Riechmann and Holliger. 1997; Deng et al. 1999) shows that the presence of the D2 domain increases specificity and that D2 has an important role in preventing F-pili independent infections. The binding of D1 to the specific receptors on the surface of the *E.coli* cell (a feature that is not F⁺-specific) is represented in **Figure 2.** This process triggers the injection of the viral genome into the bacterium (as depicted in **Figure 3**). Although loss of the D2 domain results in the formation of phage particles that can infect *E.coli* in a somewhat reduced specific manner, it appears that the level of infections from such a population of phages is significantly reduced. A chimaeric phage of the invention relies significantly for infection on the functional form of the phage coat protein and on the presence of part(s) of said phage coat protein that contribute to the infectivity of the phage. Said mutant form of said phage coat protein is mutated in the part(s) of said phage coat protein that render the phage infectious. The mutation is exemplified in, but not limited to, deletions, residue- or fragment substitutions, swaps and/or replacements by other protein fragments rendering it less infectious. Said protein fragments may or may not be related to phage coat proteins or fragments thereof, and are essentially not capable of inducing infection of the phage particle into a host cell.

In another embodiment the invention provides a phage collection comprising a chimaeric phage or an infectious phage according to the invention. Phages of the present invention are particularly useful for the generation of phage display libraries. Therefore, in a more preferred embodiment said phage collection is a phage display library. In an even more preferred embodiment said phage collection consists essentially of chimaeric phages or of infectious phages of the invention. A proteinaceous molecule, such as (random or not random) stretches of amino acids, peptides, protein parts or even entire proteins can be fused to the phage coat proteins and can form a first specific binding pair member. This fusion is typically done at the terminal ends of the coat protein. Addition typically does not affect the function of the phage coat protein. Moreover, it also often does not interfere with the function of the added moiety. Thus it is possible to generate libraries that for instance can be used to locate and to clone specific binding molecules. Such libraries can comprise peptides or larger molecules. Preferably, said larger molecules comprise a protein such as an antibody or a functional part, derivative and/or analogue thereof, such as full length heavy and/or light chains from an immunoglobulin molecule, or fragments of immunoglobulins such as Fab fragments, single chain Fv (scFv) fragments, CDR regions, sole variable regions and/or combinations of the above.

In another aspect the invention provides a method for making a phage particle which comprises the steps of providing a host cell with a first nucleic acid encoding a fusion protein, said fusion protein comprising a proteinaceous molecule fused to a functional form of a phage coat protein or to a functional part, derivative and/or analogue of said phage coat protein, providing said host cell with a second nucleic acid encoding a mutant form of said phage coat protein, said mutant form being characterized as described above and wherein less infectious may also mean non-infectious, and culturing said cell to allow assembly of said phage, said host cell otherwise or additionally comprising nucleic acid encoding at least all essential proteins, or functional equivalents of said essential proteins for the assembly of said phage particle. In a preferred embodiment the invention provides a method wherein the nucleic acid encoding at least all other proteins or functional equivalents thereof that are essential for the assembly of said phage particle in said host cell is comprised by a helper phage and said helper phage is used to deliver the nucleic acid to said host cell. In a more preferred embodiment the nucleic acid that is delivered by said helper phage also comprises said second nucleic acid encoding said mutant form of said phage coat protein. In an even more preferred embodiment said first and said second nucleic acid are separate nucleic acids that each may comprise separate unique selection markers to ensure that the host cell comprises at least one copy of each separate nucleic acid and that each comprise separate unique origins of replication to ensure no interferences during replication. In another aspect of the invention the number of possible homologous recombination events between overlapping stretches of nucleic acid sequences between the separate nucleic acids is reduced due to the use of different codons within each nucleic acid.

Using a method of the invention it is possible to generate phage particles that comprise at least two variants derived from the same coat protein. The relative number of the variants can vary. Typically, one wants to influence the relative amount of the various variants in said phage coat. To that end it is preferred that expression of said fusion protein and/or expression of said mutant form of said phage coat protein is regulatable. Preferably, this is achieved by regulating the expression of the gene encoding said phage coat protein at a transcriptional level. Thus, preferably, expression of said fusion protein and/or said mutant form of said phage coat protein is under the control of a promoter that is well controlled. Particularly advantageous is the AraC/BAD promoter. The AraC/BAD promoter is preferred because it is a promoter that is controlled in a very tight manner. This promoter is for all practical purposes silent in the presence of glucose and only slightly leaky in the absence of glucose. In addition, the activity of the promoter can be regulated very tightly by the addition of arabinose to the medium. The concentration arabinose used determines the level of protein expressed in *E.coli* cells. Therefore, optimal regulation of phage coat protein is accomplished by using this AraC/BAD promoter and by altering the culturing conditions of the host cell. The use of a promoter that is dependent on arabinose such as the AraC/BAD promoter, instead of IPTG, such as the lac-operon, prevents problems that will occur due to co-encapsidation of the helper plasmid in viral particles during helper phage synthesis. As described above, it has been shown that co-encapsidation of plasmids together with the phage genome do occur (Russel and Model. 1989; Krebber et al. 1995; Rakonjac et al. 1997). If co-encapsidation occurs with a lac driven helper plasmid it will compete with the lac driven vectors generally used for the phage display resulting in the efficient production of infectious phage particles that will not contain the g3p-X fusion product. This problem does not occur when the AraC/BAD promoter is used.

A method of the invention for the production of a phage particle is preferably used to produce a chimaeric phage according to the invention. The host can be provided with nucleic acid encoding a phage protein in any suitable way. Preferably, however, said host is provided with a helper phage according to the invention. A helper phage according to the invention comprises nucleic acid encoding other phage proteins or functional equivalents thereof that are essential for the assembly of said helper phage, said nucleic acid further encoding a mutant form of a phage coat protein, wherein said mutant form is characterized in that a phage, comprising no wild type phage coat protein from which said mutant form is derived and having a coat comprising mutant forms of said phage coat protein and no functional forms of said phage coat protein, is less infectious than a phage comprising no wild type phage coat protein from which said mutant form is derived and having a coat comprising at least one functional form of said phage coat protein and wherein said helper phage does not comprise nucleic acid encoding a functional form of said phage coat protein. With other phage proteins is meant other than said functional form of said phage coat protein and said mutant form of said phage coat protein. Nucleic acid encoding the latter may be provided to said host cell in an alternative fashion. However, said helper phage may further comprise nucleic acid encoding said functional form of said phage coat protein or said mutant form or both. Preferably, said helper phage does not comprise nucleic acid encoding said fusion protein. In this way said helper phage is uniform and may be used to produce phages that preferentially comprise nucleic acid encoding said fusion protein in the absence of nucleic acid encoding any other required helper phage protein. Thus preferably said fusion protein and said mutant form of said phage coat protein are encoded by separate nucleic acids. Preferably, each of said separate nucleic acids comprises a unique selection marker. Preferably, said separate nucleic acids comprise non-interfering origins of replication, wherein said origins of replication do not compete with one another resulting in bacterial cells that tolerate the separate nucleic acids for the generation of new phage particles.

The art teaches that it has been very difficult to generate helper phage batches, wherein said helper phages harbor nucleic acid that encodes for all essential proteins required for assembly of a phage particle in a bacterial host cell and wherein said nucleic acid lacks a gene encoding for g3p. Subsequently, the art teaches that it is difficult to produce phage libraries using such helper phages. Several difficulties are known in the art that hamper a proper generation of such helper phage batches. The present invention provides methods and means and a good combination of features such as the use of specific origins of replication, selection markers and codons in overlapping stretches of DNA, that enable the production of phage batches containing high titres of useful helper phages, that can subsequently be applied for the generation of chimaeric or infectious phages according to the invention. Therefor, in one embodiment the invention provides methods and means for the production of helper phages that carry functional forms and/or wild type forms of a phage coat protein in their coat, but that nevertheless lack nucleic acid encoding for said functional form and/or said wild type form of said phage coat protein. The invention provides a method for producing a helper phage comprising the steps of:
providing a host cell with a first nucleic acid encoding a functional form of a phage coat protein, providing said host cell with a second nucleic acid encoding a mutant form of said phage coat protein, wherein said mutant form is characterized in that a phage, comprising no wild type phage coat protein from which said mutant form is derived and having a coat comprising mutant forms of said phage coat protein and no copies of said functional form, is less infectious than a phage comprising no wild type phage coat protein from which said mutant form is derived and having a coat comprising at least one functional form of said phage coat protein, and culturing said host cell to allow assembly of said helper phage, said host cell additionally comprising nucleic acid encoding at least all other proteins or functional equivalents thereof that are essential for the assembly of said helper phage in said host cell. Preferably said method does not comprise the incorporation of a wild type form of said phage coat protein in said helper phage. Preferably said phage coat protein is the g3 protein present in the coat of most, if not all bacteriophages. Preferably, said other proteins are encoded by said second nucleic acid, wherein expression of said mutant form of said phage coat protein and/or the expression of said functional form is regulated by altering the culturing conditions of the host cell and that is preferably under the control of a controllable promoter such as the AraC/BAD promoter as described above.

A phage coat protein is said to be contributing significantly to the infectiousness of a phage when it allows, upon incorporation in a phage, said phage to infect a bacterial host in a manner comparable to a wild type version of said phage coat protein, in kind not necessarily in amount. As used herein the terms less-infectious or non-infectious refers to a phage, carrying no functional or wild type forms of a phage coat protein, preferably g3p, and that exhibits a significant diminished infectious capability as compared to the wild type phage (particle) as determinable in for instance plaque assays well known to persons skilled in the art. As used herein, the terms less- and non-infectious may also refer to a decrease in host cell specificity. In general, a non-infectious phage is unable to enter a bacterial cell as outlined for wild type phages in **Figure 2 and 3,** whereas a less infectious phage is capable of doing so with less efficiency. Preferably, a phage having mutant forms infects the host cell with an efficiency which is less than 50% compared to a phage carrying at least a functional form, more preferably less than 10%, still more preferably less than 1%, under conditions which are otherwise comparable.

In embodiments wherein said phage coat protein comprises a g3p, said mutant form preferably comprises at least a structural part, derivative and/or analogue of the D3 region. Said structural part, derivative and/or analogue comprise at least the functionality of g3p to allow assembly of stable phage particles.

A derivative of a protein as used herein comprises the same activity in kind not necessarily in amount as the protein the derivative is derived from. When the protein is a functional form of a phage coat protein the derivative comprises the same functionality in kind not necessarily in amount. When the protein is a mutant form of a phage coat protein the derivative is also a mutant form of said phage coat protein. When the protein is a mutant form of a phage coat protein that renders a phage carrying only mutant forms of said phage coat protein less infectious, the derivative also is a phage coat protein that renders a phage carrying only derivatives and/or mutant forms of said phage coat protein less infectious. Typical derivatives are proteins comprising one or more conservative amino acid substitutions. However, derivatives may also comprise insertions and/or deletions. Furthermore, derivatives may also comprise swaps of amino acids or sequences of amino acids within the same protein or between two or more related and/or unrelated proteins. An analogue of a protein as used herein comprises the same activity in kind not necessarily in amount as the protein the analogue is analogous to. When the protein is a functional form of a phage coat protein the analogue comprises the same functionality in kind not necessarily in amount.

Prior to the invention it has been difficult to generate sufficient numbers of helper phages that lack a functional g3 gene or a part, derivative and/or analogue thereof in their genome, but that nevertheless are infectious for one round of infection through *E.coli* cells. The present invention succeeds in generating such helper phages efficiently. Such helper phages carry functional forms and/or wild type g3 proteins (g3p's) on their surface, but nevertheless lack nucleic acid encoding said functional form and/or said wild type g3 protein. The invention further provides methods and means to generate libraries comprising chimaeric phages with the help of such helper phages. Preferably, phage display libraries are generated. More preferably, libraries display a large variety of single chain Fv (scFv) fragments. Using the means and methods of the invention phage libraries can be generated that contain a significant higher number of infectious phages as compared to the number of non- or less-infectious phages than were described and are present in the art. At the same time the helper phages used to produce such libraries become (through an infection round in *E.coli* cells) essentially non-infectious, because the g3 gene is not present in an infectivity-contributing form in said phages. Thus after infection of a bacterial host, said phages cannot spread to other bacterial cells except of course through division of the already infected host into daughter cells. Such libraries are therefore also provided by the invention. The generated libraries are particularly useful for panning experiments because the titers of phages per milliliter are significantly higher than was used in the art until the present invention. Moreover, libraries of the invention display less a-specific stickiness. Thus said libraries display less false positives than libraries in the art. Moreover, after one round of panning only phages that display a functional form on their surface (preferably fused to a proteinaceous molecule) can be amplified in *E.coli* cells, while phages that do not carry any functional forms of said phage coat protein but only carry mutant forms of said phage coat protein are essentially non-infectious and cannot be amplified on *E.coli* cells. Therefore, the number of remaining phages that are used for a second round of panning is significantly decreased. As a result of using the chimaeric phages of the invention present in the libraries provided by the present invention the number of panning rounds is decreased and the number of relevant binders is obtained in a much more sufficient manner as was possible before the present invention.

In another aspect the invention provides nucleic acids and helper phages comprising said nucleic acids that comprise genomic DNA sequences in which at least the domains of g3p that are responsible and contributing to infection are functionally removed. The invention also provides helper phage genomic DNA's in which the leader and at least the D3 domain are unaffected and fused together. Said nucleic acids are preferably based on VCSM13 and M13K07 genomic sequences. Due to a lack of a functional D1 domain phage particles produced by said nucleic acids are essentially non-infectious. Essentially in this context means that no spread or at least significantly less spread of said phages to other bacterial cells than the production bacterium occurs through g3p provided infectious features. This absence of spread to other bacterial cells is due to the absence of a functional form of g3p. If during production a source for wild type form or functional form of g3p is provided, produced phages can infect a bacterium. However, if said bacterium produces phages as a result of the infection then a resulting phage particle is not capable of infecting another bacterium unless again a source for infectious g3p is provided during production. A chimaeric phage or an infectious phage of the invention preferably comprises a part of g3p that ensures the generation of a stable phage particle after one round of infection in *E.coli* cells. To this end said helper phage preferably comprises a nucleic acid encoding a mutant form of said g3p. Preferably, said mutant form comprises D3 or a functional part, derivative and/or analogue thereof.

A phage display library can for instance be generated by providing a collection of bacteria with a library of nucleic acids encoding g3p fused to a range of different proteinaceous molecules and infecting said bacteria with helper phages of the invention. In a particularly preferred embodiment a library of phage display particles that is produced with these helper phages contains phages that do not carry any infectious parts of g3p on their surface and phages that carry one or two full length g3p-X fusions, next to non-infectious or less-infectious parts of g3p deletion proteins. Phages in these library mixtures that do not express g3p-X fusion proteins cannot infect bacteria anymore, since they were generated in the absence of infectious g3p parts that are not fused to X (X comprises a proteinaceous molecule or a fusion partner of interest such as immunoglobulins or fragments of immunoglobulins such as Fab fragments or scFv fragments).

In one aspect the invention provides helper phages that combine the presence of a selection marker with the presence of a bacterial origin of replication (ORI) to overcome the described problems in the production of g3-minus helper phages and subsequently for the generation of phage display libraries. The presence of such a combination ensures the production of large amounts of helper phages and/or helper genomes. g3-minus helper phages with an ORI and a resistance marker can be made from the g3-minus helper phages VCSM13 and M13K07. These helper phages, unlike M13 or R408, do contain a kanamycine resistance gene from the Tn903 transposon and a P15A ORI that are both inserted in the intergenic region of the phage genome. Another aspect of the invention is the fact that because of this resistance gene and the presence of this particular ORI, these helper phages can grow easily in large quantities, while empty, or no plasmid- or no genome-containing bacteria are removed under the selection pressure, and that no interferences occur between ORI's from the phage genome and the helper plasmid or between ORI's from the phage genome and the display library plasmids, when both nucleic acids are present in the same bacterial host cell. VCSM13 and M13K07 contain the P15A ORI. To prevent the disappearance of the helper genome or the helper vector the ORI's should not cause any interferences and therefore P15A derived ORI's are not used in the vector. For the vector applied for the production of helper phages, the ColE1 ORI was chosen to ensure that the copy number of helper plasmids can only reach low or moderate levels and to minimize the possibility of recombination, growth delay, high g3p expression levels and non-specific incorporation of the helper vector in the virus particle. Besides the features and effects mentioned above, it is also important that the helper vector for the generation of helper phages does not carry an F1 ORI to prevent the incorporation of the helper vector in the phage particle instead of the viral genome.

The invention also provides vectors enabling a regulated expression of the mutant form and/or the functional form of g3p by the use of a regulatable promoter as stated above and that furthermore contain a resistance gene that is different from the kanamycine resistance gene present in the helper genome. This complementary resistance is here provided by the betalactamase (ampicillin) gene since its product is relatively stable and ensures complete killing of bacteria that do not express the gene product.

The pBAD/gIII vector (Invitrogen) can be used as a backbone vector for the production of helper vectors of the invention. Preferably, further features of this basic helper vector are that regions of sequence homology are minimized which significantly decreases the possibility of homologous recombination.

The invention further provides the use of TOP10 and LMG host cell bacterial cells (Invitrogen) for the production of helper phages that contain a g3-minus genome but are nevertheless infectious due to the g3p present on the phages because it was delivered by the helper vector. The genotype of these bacteria ensures that they can transport arabinose into the cell but that they cannot metabolize it (genotype: araABCD- and araEFGH+). In addition, the TOP10 bacteria are recA and endA deficient which diminishes the chance of recombination and mutation. Furthermore, the TOP10 bacteria are F-, which makes them resistant to phages that might contaminate phage batches of interest.

The present invention provides further a partially deleted g3 gene that is still present in the helper phage genome to provide stable but essentially non-infectious helper phages that harbor infectious g3p's on their coat. The invention describes this partially deleted g3 gene, that is made synthetically by using synthetic primers in such a way that the functionally deleted g3 gene encodes the same protein on an amino acid level as compared to the other part of the g3 gene that is present in the same bacterium, but the codons that are used do not lead to homologous recombination events. Because the leader sequences in the different settings are very different there is no need to change these regions in the helper genome, phage display vector (with the scFv encoding genes) or helper vector. In principle it does not matter whether the g3 gene in the helper genome, in the phage display vector or helper vector has been changed as long as the two overlapping (in amino acid content) and previously homologous g3 parts that are introduced into one *E.coli* cell do not match.

The present invention describes the use of codon changes in the g3 gene for the production of helper phages that are infectious due to g3p's encoded by the helper vectors but that lack a wild type or at least an infectious g3 gene in their genome and for the production of chimaeric phages according to the invention. The use of codon changes ensures a diminished chance for homologous recombination effects that might occur during the process of helper phage generation. The invention preferably provides the use of codon changes in the g3 gene or parts thereof for the generation of phage display libraries in which the helper phage genome, that is brought into an *E.coli* cell together with nucleic acids encoding for g3p-X fusion proteins, is not homologous to the g3 gene present in the DNA encoding for the g3p-X fusion protein. These codon changes ensure that the chance for homologous recombination events in the generation of phage display libraries is significantly decreased through which the quality of these libraries and uses thereof are significantly improved.

### EXPERIMENTAL PROCEDURES

### Primers

The following primers (Genset, France) were used in the generation of the different vectors and helper phage genomic constructs. Most restriction enzymes hardly or fail to digest DNA if their corresponding palindrome is near the end of the DNA. Therefore a stretch of 8 nucleotides was added to the 5' end of all of these primers in which this stretch is an A/T rich non-hybridising 8-mer.

D3 primers
D3 BamHI Forward
5′-GGATCC TCTGGTTCCGGTGATTTGATTATG-3′
D3 BamHI Backward
5′-GGATCC AGCGGAGTGAGAATAGAAAGGAAC-3′

g3-minus primers
g3 minus HindIII Forward
5′-AAGCTT CTGCGTAATAAGGAGTCTTAATCATGC-3′
g3 minus HindIII Backward
5′-AAGCTT GTTGAAAATCTCCAAAAAAAAAGGC-3′

g3 ORF primers
g3 ORF NcoI Forward
5′-CCATGG CTGAAACTGTTGAAAGTTGTTTAGC-3′
g3 ORF XbaI Backward
5′-TCTAGA TTAAGACTCCTTATTACGCAGTATG-3′

### PCR reactions and product isolation

All PCR reactions were as a standard (except for the elongation time of the DNA synthesis cycle step) performed using the following 50 µl hot start PCR scheme and the AmpliTaq PCR kit from Perkin Elmer: 1 µl 10mM dNTP (Roche Diagnostics), 4 µl 25mM MgCl₂, 5 µl 10X PCR buffer supplied with the kit, 5 µl 2.5 µM Forward primer, 5 µl 2.5µM Backward primer, 0.3 µl 5 units/µl AmpliTaq, 10-50 ng template, sterile bidestilled water. All components were kept on ice until placing in the pre-heated PCR block. The standard program was as follows. 12 cycles of 25 sec 94°C, 52°C annealing for 25 sec, 72°C polymerisation ending with one cycle of 72°C for 7 min followed by an indefinitive storage at 4°C. The time of polymerisation for new helper genome synthesis was 12 min, and for g3 amplification and for AraC gene and AraC/BAD promoter amplification this was set at 90 sec.

All PCR products were separated on 0.5%-1% TBE agarose gels containing 100 ng/ml ethidium bromide. After imaging the desired fragments were cut out using sterile disposable chirurgical knives and isolated with Qiagen's gel purification kit according to the guided protocol.

### Ligation reactions

All ligation reactions were performed in the following reaction mixtures:
50 ng vector or helper genome
25 ng insert
4 µl 5X ligation buffer (Gibco BRL)
1 µl T4-ligase (Gibco-BRL, 200 units/µl)
sterile bidestilled water to 20 µl
The mixtures were incubated overnight at 6-10°C. Then, 30 µl sterile water, 5 µl K-acetate 3M pH 4.8 acidic acid adjusted (KAc), 1 µl glycogen 10 mg/ml and 50 µl isopropanol were added and mixed thoroughly. After 15 min of precipitation the tubes were centrifuged at maximum speed at 4°C for 10 min. The pellet was once washed with 1 ml 70% ethanol and after drying dissolved in 10 µl sterile water. Half of this volume was used for electroporation together with 50 µl competent cells.

### Sequencing

Sequencing of the clones was performed according to the instruction guide sent along with the Rhodamine BigDye terminator kit with a few adaptations. The reaction volume was scaled down to 12.5 µl. 4 ìl Qiagen mini prep purified DNA (8%) was used as template. All clones were sequenced in order to verify the correctness of the products.

### Electroporation

All bacterial strains, except those that were used for the helper phage production, were acquired from manufacturers as electroporation competent cells with the highest competence available and transformed according to the manufactures protocol using 0.1 cm cuvettes (BioRad). The production of helper phages, however, is dependent on TOP10 or LMG cells (Stratagene) containing the helper plasmid (pBAD/gIII-g3). These cells were made competent and stored at -80°C until use as follows: One colony of the bacteria was used to inoculate in 10 ml 2X TY with ampicillin (100 µg/ml) and for LMG also with tetracycline (10 µg/ml) and cultured by vigorously shaking at 30°C overnight. Next, the cultures were spun down at 3000 rpm for 5 min. The pellet was resuspended in 500 ml fresh 2X YT including the antibiotics and cultured until OD 0.5 in a 2 l Erlenmeyer flask on a shaking platform at 37°C. These cells were allowed to cool on ice-water for 45 min and centrifuged in pre-cooled buckets and rotor at 3000 rpm at 4°C for 25 min in a Sorvall centrifuge using a GLA-3000 rotor. The supernatant was discarded and the cells were slowly and carefully resuspended in 100 ml ice-cold 10% glycerol. The centrifugation and glycerol steps were repeated twice. The final pellet was taken up very carefully in 5 ml 10% ice-cold glycerol and aliquoted in pre-cooled eppendorf tubes. Next, these tubes were immersed in a mixture of ethanol and dry ice for 5 min to ensure very quick freezing of the cells. The tubes containing the electrocompetent cells were stored at -80°C until use.

### Phage production

The desired F⁺ *E.coli* strain is inoculated in 2X YT medium containing the required antibiotics and cultured at 37°C at 220 rpm until OD 0.2. The (helper-) phage is added to the culture and incubated for 30-45 min at the 37°C in a non-shaking waterbath. Then, kanamycine (50 µg/ml) is added to the cells and cells are further incubated at 220 rpm at 37°C for 30-45 min. Subsequently, this solution is spun at 3500 rpm at room temperature for 15 min. The supernatant is removed carefully and the pellet is brought to the desired volume of 2X YT medium containing all required antibiotics. Cells are cultured overnight at 30°C for a phage display library and at 37°C for regular (helper-) phages on a shaking platform.

### Titer determination

One colony of Xllblue (Stratagene) is inoculated in 5 ml 2X YT containing 10 µg tetracycline per ml (YT-T) in a 50 ml tube (Flacon) and cultured at 37°C at 220 rpm overnight. 200 µl of this culture is added to 5 ml YT-T and cultured until OD 0.2. Then, the phage stock is diluted in YT and a dilution series as required is made to determine the number of plaque forming units. For each dilution step 100 µl of the O.D. 0.2 XLlBlue culture is taken and added to 100 µl of the phages. This mixture is incubated for 25 min at 37°C in a waterbath (not shaking). The 200 µl of bacterial cells is pipetted onto a 2X YT-broth plate containing the required antibiotics. The suspension is spread using a sterile glass rod. After drying the plates they are inverted and transferred into a 37°C incubator. After overnight culture the number of colonies are counted. Each colony indicates the presence of 1 infective phage particle in the original phage solution. The number of infectious particles per ml of the analysed stock is calculated. The phage particles are ELISA tested according to the protocol supplied with anti-M13 and anti-M13-HRP conjugate (Pharmacia).

### Isolation of DNA from phages

An overnight phage culture is grown as described above. If a large scale isolation of DNA was required the BioRad Plasmid Maxi Prep kit was used according to the manufactures instructions, except for the elution step which is done with 10 mM Tris pH8.5 at 65°C for 10 min. Small or medium scale isolations were performed using Qiagen's mini-prep kit according to the instruction supplied with the kit except for the elution step. The elution step here was performed at 65°C for 10 min.

### PEG precipitation

The medium containing bacteria and phages is collected in 450 ml buckets. The mixture is spun in a pre-cooled Sorvall centrifuge using a GSA-3000 rotor at 8000 rpm for 20 min. Then, 90 ml 20% PEG/2.5 M NaCl is pipetted into clean 450 ml buckets. 360 ml of the supernatant of the centrifuged medium containing the phages is brought into the PEG containing buckets and mixed well. The mixture is set on ice water for 2 h or overnight in the fridge. The precipitate is pelleted by centrifugation in a pre-cooled Sorvall centrifuge at 8000 rpm for 20 min. The supernatant is decanted and the buckets are left to drip out for 5 min in order to remove as much Precipitation buffer as possible. Subsequently, 32 ml PBS/1% bovine serum albumin (BSA) is added to the 450 ml buckets containing the pelleted phages and buckets are rotated on a bottle roller for 15 min. The solution is transferred to a SS-34 compatible centrifuge tube and spun in a pre-cooled Sorvall centrifuge containing a SS-34 rotor (or equivalent equipment) for 25 min at 13,000 rpm. This step removes all kind of debris and small bacteria. In the meantime, the plunger is removed from a 50 ml syringe and attached to a 0.45 µM filter (Whatmann). The centrifuged supernatant is transferred into the syringe and pushed through the filter. This step removes all small bacteria and other cells. 8 ml 20% PEG/2.5 M NaCl is added and mixed well. The tubes are set on ice for 1 h. The high speed centrifugation step is repeated as described above. The supernatant is decanted and the tube is let to drip out on a paper towel for 5 min. The phage pellet is dissolved in 5 ml PBS/1% BSA. Then, 5 ml 100% glycerol is added to the phage solution and mixed well. Phages are stored at -20°C. Typically, the solution contains approximately 2 to 5x10¹³ infectious phage particles per ml.

### EXAMPLES

To illustrate the invention, the following examples are provided, not intended to limit the scope of the invention.

### Example 1.

### Cloning of the pBAD/gIII-g3 helper vector.

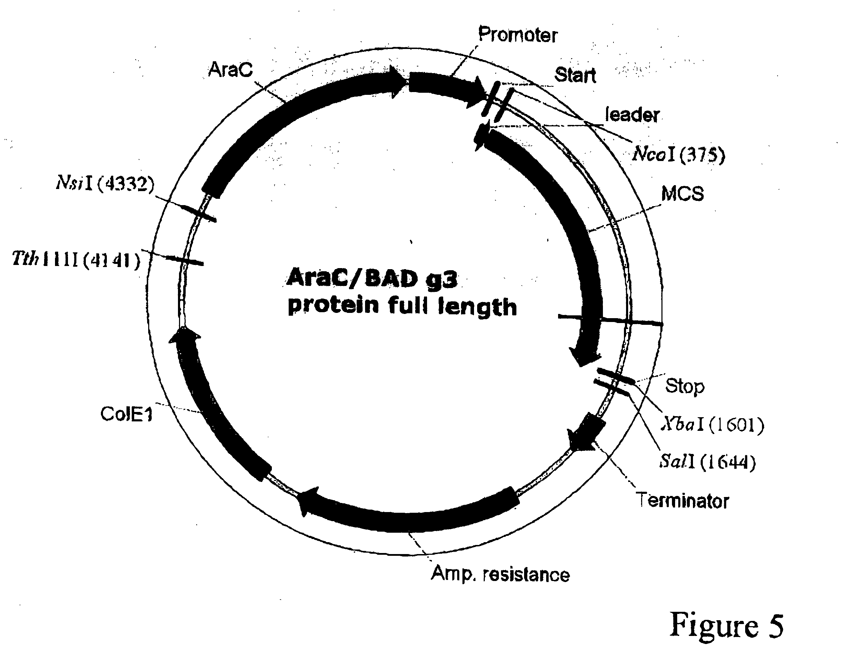

The full Open Reading Frame (ORF) of the g3 gene was generated by using M13KO7 (Gibco-BRL) DNA as a template in a standard PCR reaction together with g3 ORF NcoI Forward and g3 ORF XbaI Backward primers. The purified PCR product and the pBAD/gIII vector were both digested with NcoI (NEB) and XbaI (Roche Diagnostics) simultaneously in buffer H (Roche Diagnostics) for 4 h at 37°C. After ligation, isolation, electroporation in TOP10 (Stratagene) and LMG (Stratagene) cells, two correct clones were selected by sequencing and grown on a large scale followed by the isolation of the DNA. The DNA was reprecipitated with 70% ethanol and in the presence of KAc and the pellet was washed twice with 70% ethanol. After drying the DNA was solved in sterile bi-destilled water and stored at -20 °C until use. The resulting plasmid is depicted in **Figure 5**. This helper vector contains the full sized g3 gene under the control of the AraC/BAD promoter, ampicillin resistance gene and a ColE1 ORI as most important features.

### Example 2.

### Cloning of the g3 minus helper phage genome g3⁻-HP.

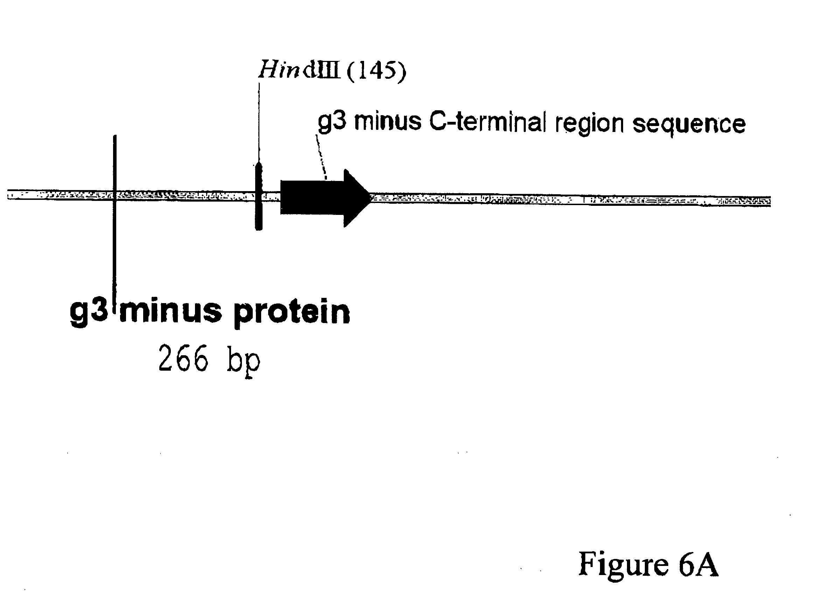

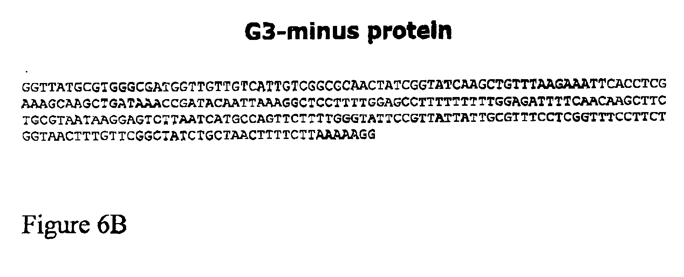

The use of g3 minus HindIII Forward and g3 minus HindIII Backward primers and M13KO7 and VCSM13 as templates in a standard PCR reaction resulted in the formation of a PCR product that contained HindIII sites at both ends of the DNA. After separation, gel isolation and purification, digestion with HindIII (Roche Diagnostics) and re-purification of the DNA, the product was self-ligated under standard ligation conditions and electroporated into XL1Blue cells (Stratagene). The transformed cells were resuspended in 5 ml 2TY medium and cultured shaking at 37°C for 1 h. Kanamycin was added to an end-concentration of 50 µg/ml and the cells were allowed to grow at the same conditions for another 5 h. The culture was centrifuged at 3000 rpm for 15 min and the supernatant passed through a 0.22 µM filter to remove bacteria. At the same time, a culture of exponentially growing XL-1 Blue bacteria was prepared. Fractions of the filtrate (50-1000 µl), containing phage particles, were added to 5 ml of XL-1 Blue bacteria and incubated at 37°C for 30 min without shaking. The culture was centrifuged again, the supernatant discarded and the cells were plated on 2X YT-K-T plates and transferred into an incubator at 37°C for overnight growth. Eight correct clones that lack the g3 ORF (checked through the BamHI site) and include the introduced HindIII site were isolated and used for g3-less helper phage production in the presence of the pBAD/gIII-g3 helper plasmid. Only two clones that were able to form phages in the presence of the helper plasmid were kept. From these clones a large quantity of DNA was isolated and stored for further experiments. The obtained g3-minus helper phage genome is depicted schematically in **Figure 6A,** while the correct sequence of this construct surrounding the HindIII and BsrI sites is depicted in **Figure 6B.**

### Example 3.

### Cloning of a helper phage genome with a partially deleted g3 gene.

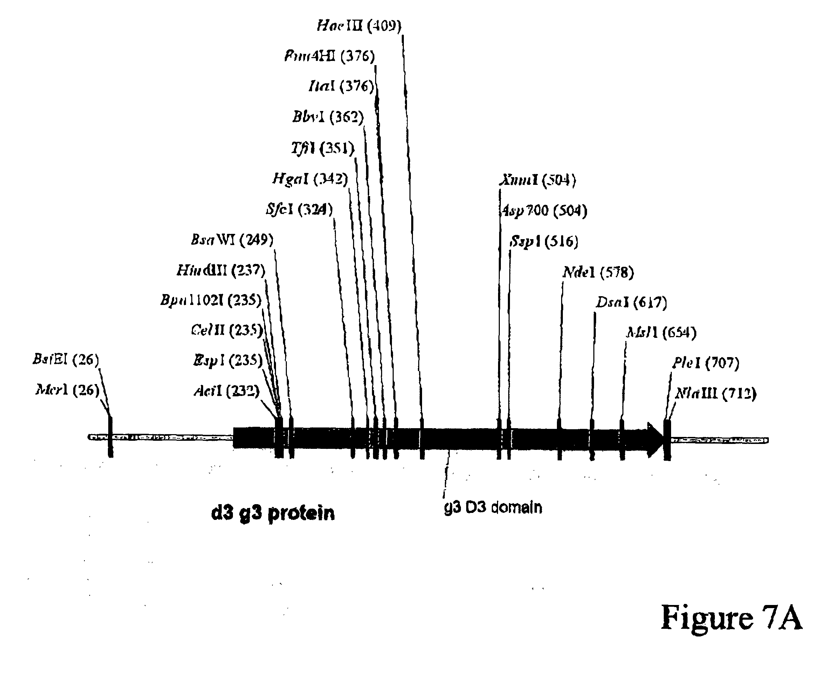

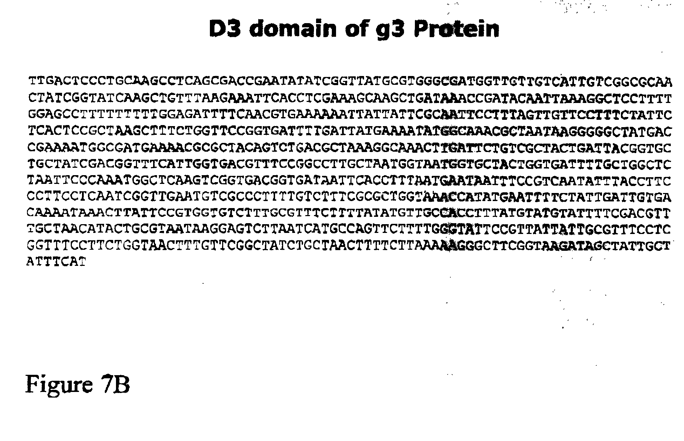

The construction of helper phage genome that express only the D3 part of the g3 gene was comparable to the above described g3-less helper phages with the exception that the primers used were D3 BamHI Forward and D3 BamHI Backward primers in order to generate the new genome. All other procedures were the same as for the g3-less procedure, except for the use of BamHI instead of HindIII. In the end, the DNA of two correct clones was kept and stored at -20°C. The final construct of the helper phage genome that still expresses the part of the g3 gene that does not contribute to the infectiousness of the phage particle is depicted in **Figure 7A,** while the sequence surrounding the PCR product insert in the genome is depicted in **Figure 7B.**

### Example 4.

### Production of infectious helper phages that do not carry a gene encoding the wild type g3p.

The procedures for generating g3-less and generating partially deleted g3 (or D3 expressing) helper phages are identical. Frozen competent TOP10 or LMG cells that contain the pBAD/gIII-g3 helper plasmid were electroporated using 100 ng helper phage DNA. After recovery the cells were transferred into 4X 250 ml 2X YT-K-A medium supplemented with 0.05% arabinose (Sigma). Phages were produced during overnight culture at 37°C and vigorously shaking. The next day the phages were purified and stored according to the standard procedures of precipitation and storage that were described supra. The number of infectious particles and the number of phages were determined by titration and ELISA procedures that were also described supra. For g3-less helper phages approximately 5x10¹¹ infectious particles were synthesized while for D3 approximately 5x10¹³ infectious phages were formed using these procedures.

### Example 5.

### Amplification and harvesting of phage display libraries containing infectious phages carrying g3p-scFv fusions and non-infectious helper phages, using g3 minus and partially g3 deleted helper phages.

A frozen library was inoculated as follows. In general approximately 5-10 µl concentrated stocks were inoculated in 25 ml 2X YT containing the required antibiotics and 5% glucose and grown at 37°C with vigorously shaking. At OD 0.3-0.4 (after about 2-3 h), a 500-1000 fold excess of helper phage was added. The medium containing the helper phages and bacteria was incubated in a water bath at 37°C without shaking for 25 min. Removal of death cells and excess of phage particles was realized after a centrifugation step at 3000 rpm for 15 min. The pellet was resuspended in 250 ml 2X YT with antibiotics but without glucose and grown at 30°C with good aeration overnight. The next day the formed phages were isolated and stored using the standard PEG/NaCl procedure.

### Example 6.

### Codon usage in g3 and the partially deleted g3 gene (D3) to prevent homologous recombination during helper phage production and library amplification.

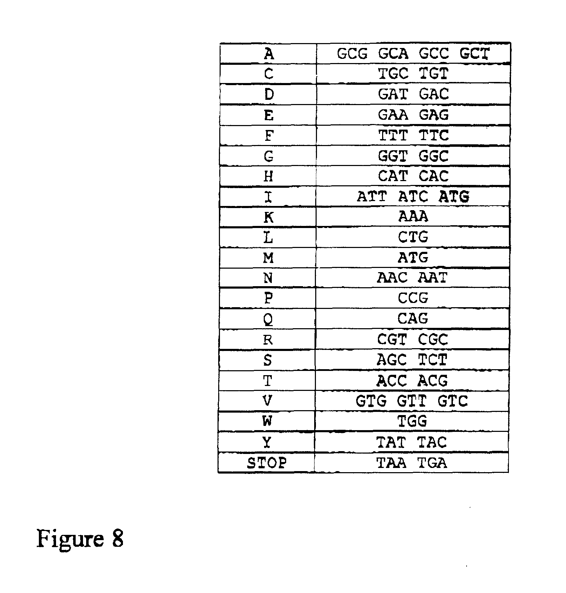

In order to prevent possible recombinations between a genomic nucleic acid encoding the helper phage g3 protein region (or a part thereof such as the D3 domain) and other nucleic acids (like the phage display vector and the AraC/BAD helper vector), a series of helper phages are designed that contain changed codons within the g3p region. Newly translated g3p's are identical to the wild type g3 protein or protein part (D3). Due to these changes, g3-ORF coding DNA domains cannot or barely recombine with the phage display vectors or the AraC/BAD-g3 helper vector. The codons that are used to generate non-homologous g3 genes are depicted in **Figure 8** and are optimal for the *E.coli* transcription machinery. PCR generation of helper phage genomes (VCSM13, M13K07, D3, g3-minus or AraC/BAD) with g3-leader backward and g3 end forward primers with NotI restriction sites ensure the generation of PCR products containing all helper phage components and genes, except for the g3 ORF. New g3 regions are constructed with overlapping primers and are inserted in helper phages. The PCR-generated g3p or parts thereof are digested with NotI and ligated in the NotI digested PCR generated helper phage genome. After transformation and selection of correct helper genomes (with a new g3 gene), helper phages are grown as described.

### Example 7.

### Selection of thyroglobulin-interacting phages using a library amplified with helper phages comprising only the D3 part of g3p in their genome.

In order to validate the D3 helper phages in standard phage selections, a selection was performed using an antibody phage display library that was amplified using the D3 helper phages as described above. Procedures that were used were essentially as described by De Kruif et al. (1995a). Briefly, thyroglobulin was coated to a plastic tube. The tube was blocked in PBS containing 2% milk (MPBS) whereafter the antibody phage display library, also blocked in MPBS, was added to the tube. The phages were allowed to bind for 2 h, whereafter non-binding phages were removed by washing the tube in PBS containing 0.1% Tween-20 as described by De Kruif et al. (1995a). The binding phages were eluted in 50 mM Glycin/HCl pH 2.2 (10 min at RT) and used to infect freshly grown XL-1 Blue bacteria. The bacteria were plated on 2TY agar plates containing the appropriate antibiotics and glucose, incubated overnight at 37°C and used to prepare an enriched phage display library; phage D3 was again used as helper phage. The procedure was repeated once, whereafter individual *E.coli* colonies were used to prepare monoclonal phage antibodies. These monoclonal phage antibodies were tested in ELISA for their ability to bind specifically to the thyroglobulin antigen. Results show that after two rounds of selection 25/46 colonies show positive binding to thyroglobulin. Previously we found that by using a general VCS-M13 as a helper phage, at least 3 rounds of selection were required to obtain specific binders in this selection format.

### Example 8.

### Selection of phages interacting with myeloma cells using a library amplified with helper phages comprising only the D3 part of g3p in their genome.

In order to validate the D3 helper phages in selections on intact cells, a selection was performed using an antibody phage display library that was amplified using the D3 helper phage. Procedures that were used were essentially as described by De Kruif et al. (1995a and 1995b). Briefly, myeloma cells (AML, CD33+, CD34+) were obtained from the blood of a patient undergoing treatment at the Utrecht University Hospital (The Netherlands). 0.5 ml phage library was added to 3 ml RPMI medium containing 10% FCS (RPMIS) and incubated on ice for 15 min. The myeloma cells were added and the cell suspension was rotated at 4°C for 2 h. The cells were washed 5 times in 50 ml ice-cold RPMIS whereafter the binding phages were eluted (in 50 mM Glycin/HCl pH 2.2 for 10 min at RT) and used to infect freshly grown XL-1 Blue bacteria. The bacteria were plated on 2TY agar plates containing the appropriate antibiotics and glucose, incubated overnight at 37°C and used to prepare an enriched phage display library. Again, the D3 expressing helper phages were used as helper. The procedure was repeated once, whereafter individual *E.coli* colonies were used to prepare monoclonal phage antibodies. These monoclonal phage antibodies were tested in FACS procedures for their ability to bind myeloma cells. Results show that 23 out of 41 clones tested bound specifically to epitopes expressed on the myeloma cells. Generally, three or more rounds of selection are required to obtain similar numbers of binding phages using identical procedures, with the exception of using VCS-M13 helper phages instead of the helper phages described by the invention.

### SHORT DESCRIPTION OF THE FIGURES

**Figure 1.** Schematic representation of the g3 protein (g3p) present in the coat of M13 phages. The D3 domain of g3p is attached to the single stranded DNA inside the particle via the g8 protein (g8p), while the D1 and D2 domains interact with each other outside the particle and can be used for fusion with for example scFv.

**Figure 2.** Schematic representation of the interaction between the D2 domain of g3p with the F-pilus on the surface of *E.coli,* with a subsequent interaction of the D1 domain with other components of the bacterial surface.

**Figure 3.** Schematic representation of the interaction of the D2 domain of g3p with the F-pilus (left) and the D1 domain of g3p with the TolA receptor (see **Figure 2**) and the subsequent entry (right) of the phage genome into the cytoplasm of the bacterium.

**Figure 4.** Schematic representation of a wild type phage expressing five g3p's on its infectious end (left) and a recombinant phage expressing four wild type g3p's and one g3p-X fusion protein on its infectious end (right). The recombinant phage also harbors the genetic information of the fusion protein present on the surface.

**Figure 5.** Schematic representation of the pBAD/gIII-g3 helper vector harboring the full length g3 gene under the control of the AraC/BAD promoter and further harboring an ampicillin resistance and a ColE1 origin of replication (ORI).

**Figure 6.** (A) Schematic representation of the helper phage genome deleted for the open reading frame (ORF) of the g3 gene. The arrow indicates the 3' end of the gene that remained after cloning procedures. (B) Sequence of the part of the helper phage genome that surrounds the position of the g3 deletion depicted in (A).

**Figure 7.** (A) Schematic representation of the helper phage genome deleted for the part of the g3 gene that contributes to the infectivity of the phage. The arrow indicates the D3 part of the g3 gene that encodes the carboxy-terminal part of the g3 protein enabling the generation of stable, but essentially non-infectious helper phages. (B) Sequence of the nucleic acid that is shown in (A).

**Figure 8.** Codon usage adaptation in the g3 gene and the D3 domain to prevent homologous recombination during helper phage production and during phage display library amplification. The left panel shows the obtained one-letter coded amino acids and the right panel shows the optimal codons.

### REFERENCES

Balint RF and Larrick JW (1993) Antibody engineering by parsimonious mutagenesis. Gene 137:109
Barbas CF, Hu D, Dunlop N, Sawyer L, Cababa D, Hendry RM, Nara PL, Burton DR (1994) In vitro evolution of a neutralizing human antibody to human immunodeficiency virus type 1 to enhance affinity and broaden strain cross-reactivity. Proc Natl Acad Sci USA. 91:3809
Bass SH, Greene R and Wells JA (1990) Hormone phage: An enrichment method for variant proteins with altered binding properties. Proteins 8:309-314
Beekwilder J, Rakonjac J, Jongsma M, Bosch D (1999) A phagemid vector using the E.coli phage shock promoter facilitates phage display of toxic proteins. Gene 288:23-31
Berek C, and Milstein C (1987) Mutation drift and repertoire shift in the maturation of the immune response. Immunol Rev 96:23
Burton DR and Barbas CF (1994) Human antibodies from combinatorial libraries. Adv Immunol 57:191
Crissman JW and Smith GP (1984) Gene-III protein of filamentous phage: evidence for a carboxyl-terminal domain with a role in morphogenesis. Virology 132:445-455
Cwirla SE, Peters EA, Barrett RE and Dower WJ (1990) Peptides of phage: A vast library of peptides for identifying ligands. Proc Natl Acad Sci USA 87:6378-638
De Kruif J, Boel E and Logtenberg T (1995a) Selection and application of human single chain Fv antibody fragments from a semi-synthetic phage antibody display library with designed CDR3 regions. J Mol Biol 248: 97-105
De Kruif J, Terstappen L, Boel E and Logtenberg T (1995b) Rapid selection of cell subpopulation-specific human monoclonal antibodies from a synthetic phage antibody library. Proc Natl Acad Sci USA 92:3938-3942
Deng LW, Malik P, Perham RN (1999) Interaction of the globular domains of pIII protein of filamentous bacteriophage fd with the F-pilus of Escherichia coli. Virology 253:271-277
Devlin JJ, Panganiban LC and Devlin PE (1990) Random peptide libraries: A source of specific protein binding molecules. Science 249:404-406
Dueòas M, Borrebaeck CA (1995) Novel helper phage design: intergenic region affects the assembly of bacteriophage and the size of antibody libraries. FEMS microbiology letters 125:317-322
Felici F, Luzzago A and Cortese R (1993) Mimicking of discontinuous epitopes by phage displayed proteins. II. Selection of clones recognized by a protective monoclonal antibody against the *Bordetella pertussis* toxin from phage peptide libraries. Gene 128:21-27
Hawkins RE, Russel SJ, Winter G (1992) Selection of phage antibodies by binding affinity: mimicking affinity maturation. J Mol Biol 226:889
Holliger P, Riechmann L (1997) A conserved infection pathway for filamentous bacteriophage is suggested by the structure of the membrane penetration domain of the minor coat protein g3p from phage fd. Structure 5:265-275
Hoogenboom HR (1994) Designing and optimizing library selection strategies for generating high-affinity antibodies. Trends in Biotechnol. 15:62
Krebber C, Spada S, Desplancq D, Pluckthun A (1995) Co-selection of cognate antibody-antigen pairs by selectively-infective phage. FEBS Lett. 377:227-31
Kristensen P, Winter G (1998) Proteolytic selection for protein folding using filamentous bacteriophage. Folding and Design 3:321-328
Low NM, Holliger PH, Winter G (1996) Mimicking somatic hypermutation: affinity maturation of antibodies displayed on bacteriophage using a bacterial mutator strain. J Mol Biol 260:359
Lubkowski J, Hennecke F, Pluckthun A, Wlodawer A (1998) The structural basis of phage display elucidated by the crystal structure of the N-terminal domains of g3p. Nat Struct Biol 5:140-147
Lubkowski J, Hennecke F, Pluckthun A, Wlodawer A (1999) Filamentous phage infection: crystal structure of g3p in complex with its coreceptor, the C-terminal domain of TolA. Structure 7:711-722
Luzzago A, Felici F, Tramontano A, Pessi A and Cortese R (1993) Mimicking of discontinuous epitopes by phage displayed proteins. I. Epitope mapping of human H ferritin using a phage display library of constrained peptides. Gene 128:51-57
Lopez J and Webster RE (1983) Morphogenesis of filamentous bacteriophage f1: orientation of extrusion and production of polyphage. Virology 127:177-193
Model P, Jovanovic G, Dworkin J. (1997) The Escherichia coli phage shock protein (psp) operon. Mol Microbiol 24:255-261
Nelson FK, Friedman SM and Smith GP (1981) Filamentous phage DNA cloning vectors: A noninfective mutant with a nonpolar deletion in gene III. Virology 108:338-350
Pratt D, Tzagoloff H and Beaudoin J (1969) Conditional lethal mutants of the small filamentous coliphage. II. Two genes for coat proteins. Virology 39:42-53
Rakonjac J, Jovanovic G, Model P. (1997) Filamentous phage infection-mediated gene expression: construction and propagation of the gIII deletion mutant helper phage R408D3. Gene 198:99-103
Rakonjac J, Model P. (1998) Roles of pIII in filamentous phage assembly. J Mol Biol 282:25-41
Riechmann L, Holliger P (1997) The C-terminal domain of TolA is the coreceptor for filamentous phage infection of E. coli. Cell 90:351-360
Russel M, Model P (1989) Genetic analysis of the filamentous bacteriophage packaging signal and of the proteins that interact with it. J Virol 63:3284-3295
Smith GP (1985) Filamentous fusion phage: Novel expression vectors that display cloned antigens on the surface of the virion. Science 228:1315-1317
Spada S, Krebber C and Pluckthun A (1997) Selectively infective phages (SIP). Biol Chem 378:445-456
Vaughan TJ, Osbourn JK, Tempest PR (1998) Human antibodies by design. Nat Biotechnol 16:535
Winter G, Milstein C (1991) Man-made antibodies. Nature 349:293
Yang W-P, Green K, Pinz-Sweeney S, Briones AT, Burton DR, Barbas CF (1995) CDR walking mutagenesis for the affinity maturation of a potent human ant-HIV-1 antibody into the picomolar range. J Mol Biol 254:392

## Claims

1. A chimaeric phage having a coat comprising a mixture of proteins, said mixture comprising a fusion protein wherein a proteinaceous molecule is fused to a functional form of a phage coat protein, said mixture further comprising a mutant form of said phage coat protein, wherein said mutant form is **characterized in that** a phage, comprising no wild type phage coat protein from which said mutant form is derived and having a coat comprising said mutant form and no copies of said functional form, is less infectious than a phage, comprising no wild type phage coat protein from which said mutant form is derived and having a coat comprising said mutant form and at least one copy of said functional form.

2. A chimaeric phage according to claim 1, wherein said phage coat protein is the g3 protein.

3. A chimaeric phage according to claim 2, wherein said mutant form comprises a mutation in the D1 and/or the D2 region of said g3 protein.

4. A chimaeric phage according to claim 3, wherein said mutation comprises a deletion of substantially all of said D1 and said D2 region of said g3 protein.

5. A chimaeric phage according to any one of claims 1-4 comprising a nucleic acid encoding said fusion protein.

6. A chimaeric phage according to any one of claims 1-5, wherein said chimaeric phage is derived from a M13, M13K07, VCSM13 or R408 phage.

7. A chimaeric phage according to any one of claims 1-6, wherein said proteinaceous molecule comprises a peptide, a protein or a part, analogue or derivative thereof.

8. A chimaeric phage according to any one of claims 1-6, wherein said proteinaceous molecule comprises an antibody, a Fab fragment, a single chain Fv fragment, a variable region, a CDR region, an immunoglobulin or a functional part thereof.

9. A chimaeric phage having a coat comprising a mixture of proteins, said mixture comprising a fusion protein wherein a proteinaceous molecule is fused to a phage coat protein, or to a fragment or derivative thereof, and wherein said fusion protein is functional so as to render the chimaeric phage infectious, said mixture further comprising a mutant form of said phage coat protein, wherein said mutant form is **characterized in that** a phage, comprising no wild type phage coat protein from which said mutant form is derived and carrying said mutant form and no copies of said fusion protein, is less infectious than a phage, comprising no wild type phage coat protein from which said mutant form is derived and carrying in addition to said mutant form at least one copy of said fusion protein.

10. A chimaeric phage according to claim 9, wherein said mutant form is **characterized in that** a phage, comprising no wild type phage coat protein from which said mutant form is derived and carrying said mutant form and no copies of said fusion protein is non-infectious.

11. A chimaeric phage according to any one of claims 1-10, wherein said mutant form is further **characterized in that** a phage, having a coat comprising said mutant form in the presence or absence of copies of said functional form, is stable.

12. An infectious phage containing at least one copy of a mutant form of a phage coat protein, wherein said mutant form has lost the ability to mediate infection of a natural host by said infectious phage.

13. A phage collection comprising a chimaeric phage according to any one of claims 1-11 or an infectious phage according to claim 12.

14. A phage collection according to claim 13, wherein said phage collection is a phage display library.

15. A phage collection consisting essentially of chimaeric phages according to any one of claims 1-11 or of infectious phages according to claim 12.

16. A method for producing a phage particle comprising the steps of:
- providing a host cell with a first nucleic acid encoding a fusion protein, said fusion protein comprising a proteinaceous molecule fused to a functional form of a phage coat protein,
- providing said host cell with a second nucleic acid encoding a mutant form of said phage coat protein, said mutant form being **characterized in that** a phage, comprising no wild type phage coat protein from which said mutant form is derived and having a coat comprising said mutant form and no copies of said functional form, is less infectious than a phage comprising no wild type phage coat protein from which said mutant form is derived and having a coat comprising at least one copy of said functional form, and wherein said host cell comprises an additional nucleic acid sequence encoding at least all other proteins or functional equivalents thereof, that are essential for the assembly of said phage particle in said host cell, and
- culturing said host cell to allow assembly of said phage particle.

17. A method according to claim 16, wherein expression of said fusion protein and/or said mutant form is regulatable by altering the culturing conditions of said host cell.

18. A method according to claim 16 or 17, wherein expression of said fusion protein and/or said mutant form is under the control of a regulatable promoter.

19. A method according to claim 18, wherein said regulatable promoter comprises the AraC/BAD promoter or a functional equivalent thereof.

20. A method to any one of claims 16-19, wherein said additional nucleic acid sequence is provided by a helper phage to said host cell.

21. A method according to claim 20, wherein said helper phage comprises said second nucleic acid.

22. A method according to any one of claims 16-20, wherein said fusion protein and said mutant form are encoded by separate nucleic acids each comprising a unique selection marker.

23. A method according to claim 22, wherein said separate nucleic acids each comprises a unique origin of replication.

24. A method according to claim 22 or 23, wherein said separate nucleic acids each comprises codons that essentially do not render a homologous recombination event between said separate nucleic acids.

25. A method according to any one of claims 16-24, wherein said phage particle is a chimaeric phage according to any one of claims 1-11 or an infectious phage according to claim 12.

26. A helper phage comprising nucleic acid encoding phage proteins or functional equivalents thereof that are essential for the assembly of said helper phage, said nucleic acid further encoding a mutant form of a phage coat protein, wherein said mutant form is **characterized in that** a phage, comprising no wild type phage coat protein from which said mutant form is derived and having a coat comprising said mutant form and no copies of a functional form of said phage coat protein, is less infectious than a phage, comprising no wild type phage coat protein from which said mutant form is derived and having a coat comprising at least one copy of said functional form, wherein said functional form is **characterized in that** it renders a phage particle carrying said functional form in its coat infectious, and wherein said helper phage does not comprise nucleic acid encoding said functional form.

27. A helper phage according to claim 26, wherein said phage coat protein is the g3 protein.

28. A helper phage according to claim 27, wherein said mutant form comprises a mutation in the D1 and/or the D2 region of said g3 protein.

29. A helper phage according to claim 28, wherein said mutation comprises a deletion of substantially all of said D1 and said D2 region of said g3 protein.

30. A helper phage according to any one of claims 26-29, wherein said mutant form is further **characterized in that** a phage, having a coat comprising said mutant form in the presence or absence of a copy of said functional forms, is stable.

31. A method for producing a helper phage comprising the steps of:
- providing a host cell with a first nucleic acid encoding a functional form of a phage coat protein,
- providing said host cell with a second nucleic acid encoding a mutant form of said phage coat protein, wherein said mutant form is **characterized in that** a phage, comprising no wild type phage coat protein from which said mutant form is derived and having a coat comprising said mutant form and no copies of said functional form, is less infectious than a phage, comprising no wild type phage coat protein from which said mutant form is derived and having a coat comprising at least one copy of said functional form, wherein said host cell comprises an additional nucleic acid sequence encoding at least all other proteins or functional equivalents thereof that are essential for the assembly of said helper phage in said host cell, and
- culturing said host cell to allow assembly of said helper phage.

32. A method according to claim 31, wherein said other proteins or functional equivalents thereof that are essential for the assembly of said helper phage in said host cell are encoded by said second nucleic acid.

33. A method according to claim 31 or 32, wherein expression of said functional form and/or said mutant form is regulatable by altering the culturing conditions of said host cell.

34. A method according to any one of claims 31-33, wherein expression of said functional form and/or said mutant form is under the control of a regulatable promoter.

35. A method according to claim 34, wherein said regulatable promoter comprises the AraC/BAD promoter or a functional equivalent thereof.

36. A method according to any one of claims 31-35, wherein said phage coat protein is the g3 protein.

37. A method according to claim 36, wherein said mutant form comprises a mutation in the D1 and/or the D2 region of said g3 protein.

38. A method according to claim 37, wherein said mutation comprises a deletion of substantially all of said D1 and said D2 region of said g3 protein.

39. A method according to any one of claims 31-38, wherein said first nucleic acid and said second nucleic acid each comprises a unique selection marker.

40. A method according to any one of claims 31-39, wherein said first nucleic acid and said second nucleic acid each comprises a unique origin of replication.

41. A method according to any one of claims 31-40, wherein said first nucleic acid and said second nucleic acid comprise codons that essentially do not render a homologous recombination event between said first nucleic acid and said second nucleic acid.

42. A method according to any one of claims 31-41, wherein said helper phage is a helper phage according to any one of claims 26-30.

43. A method for the enrichment of a first binding pair member in a repertoire of first binding pair members selected from the group consisting of an antibody, an antibody fragment, a single chain Fv fragment, a Fab fragment, a variable region, a CDR region, an immunoglobulin or a functional part thereof, said first binding pair member being specific for a second binding pair member, comprising the steps of
- contacting a phage collection according to any one of claims 13-15 with material comprising said second binding pair member under conditions allowing specific binding,
- removing non-specific binders, and
- recovering specific binders, said specific binders comprising said first binding pair member.

44. A method according to claim 43 comprising the additional steps of
- recovering from a phage a DNA sequence encoding said first specific binding pair member,
- subcloning said DNA sequence in a suitable expression vector, and
- expressing said DNA sequence in a suitable host, and
- culturing said suitable host under conditions whereby said first specific binding pair member is produced.

45. A nucleic acid molecule comprising a sequence encoding a mutant form of a phage coat protein, said mutant form being **characterized in that** a phage,
comprising no wild type phage coat protein from which said mutant form is derived and having a coat comprising said mutant form and no functional form of said phage coat protein, is less infectious than a phage, comprising no wild type phage coat protein from which said mutant form is derived and having a coat comprising said mutant form and at least one copy of said functional form of said phage coat protein, wherein said functional form is **characterized in that** it renders a phage carrying said functional form in its coat infectious.
